# EUROPEAN PATENT SPECIFICATION

(11) **EP 3 908 675 B1**
(45) Date of publication and mention of the grant of the patent: **09.12.2026**
(21) Application number: 19828301.2
(22) Date of filing: 19.12.2019
(51) Int. Cl.: C12Q 1/6834, C12Q 1/6876

(54) **OLIGONUCLEOTIDE DEPOSITION ONTO POLYPROPYLENE SUBSTRATES**
ABSCHEIDUNG VON OLIGONUCLEOTIDEN AUF POLYPROPYLENSUBSTRATEN
DÉPÔT DES OLIGONUCLÉOTIDES SUR DES SUBSTRATES POLYPROPYLENE

(30) Priority: 08.01.2019 GB 201900260
(43) Date of publication of application: 17.11.2021
(73) Proprietor: QuantuMDx Group Limited, Newcastle upon Tyne, Tyne and Wear NE1 2JQ (GB)
(72) Inventor: HANNANT, Jennifer, Newcastle upon Tyne Tyne and Wear NE1 2JQ (GB); FLEMING, Jodie Leanne, Newcastle upon Tyne Tyne and Wear NE1 2JQ (GB)
(74) Representative: Definition IP Limited
(86) International application number: PCT/GB2019/053639
(87) International publication number: WO 2020/144456

(56) References cited:
- WO-A2-00/70088
- US-A- 5 554 501
- US-A1- 2001 018 513
- P. KUMAR ET AL: "A Rapid Method for the Construction of Oligonucleotide Arrays", BIOCONJUGATE CHEMISTRY, vol. 14, no. 3, 1 May 2003 (2003-05-01), pages 507 - 512, XP055084967, ISSN: 1043-1802, DOI: 10.1021/bc025646q
- SITUMA C ET AL: "Fabrication of DNA microarrays onto poly(methyl methacrylate) with ultraviolet patterning and microfluidics for the detection of low-abundant point mutations", ANALYTICAL BIOCHEMISTRY, ELSEVIER, AMSTERDAM, NL, vol. 340, no. 1, 1 May 2005 (2005-05-01), pages 123 - 135, XP022593929, ISSN: 0003-2697, [retrieved on 20050328], DOI: 10.1016/J.AB.2005.01.044
- SITUMA C ET AL: "Merging microfluidics with microarray-based bioassays", BIOMOLECULAR ENGINEERING, ELSEVIER, NEW YORK, NY, US, vol. 23, no. 5, 1 October 2006 (2006-10-01), pages 213 - 231, XP028041926, ISSN: 1389-0344, [retrieved on 20061001], DOI: 10.1016/J.BIOENG.2006.03.002
- KIRAN KUMAR GOLI: "Functional Coatings Based on Denaturation and Adsorption of Proteins", 9 May 2012 (2012-05-09), XP055464600, ISBN: 978-1-267-50543-9, Retrieved from the Internet <URL:https://repository.lib.ncsu.edu/bitstream/handle/1840.16/7785/etd.pdf?sequence=2&isAllowed=y> [retrieved on 20180404]

## Description

### Technical Field

The present invention relates to a method of immobilising an oligonucleotide on a polypropylene surface. The invention has utility in biomolecular assays, and in particular in hybridisation techniques where it can be used to attach single-stranded deoxyribonucleic acid (ssDNA) probes to the surface of polypropylene components such as microfluidic cassettes and plugs.

### Background

Microfluidic cassettes, or microfluidic flow cells, have a microfluidic structure in which one or more fluid channels are formed. The fluid channel typically comprises a flowable carrier medium, with which reagents or probes, housed in microchannels or chambers, or immobilised on the surface, are brought into contact for the purposes of analysis or testing. In most microfluidic systems, it is necessary to optimise flow of the carrier through the channels to ensure efficient throughput. This can be manipulated by careful selection of the material making up the microfluidic flow cell or components thereof.

While traditionally composed of silicon, and then glass, the use of polymeric materials in microfluidic applications has increased in recent years.

Kumar et al., Bioconjugate Chemistry, Vol. 14(3); 2003, pp. 507-512, discloses a method for the construction of oligonucleotide arrays on surfaces. The method involves the generation of hydroxyl functionalities on commonly used surfaces for construction of oligonucleotide arrays followed by their activation with trifluoroethanesulfonyl chloride (tresyl chloride). The activated surface in the subsequent reaction is used to covalently immobilise oligonucleotides in regioselective fashion to create an oligonucleotide array.

WO00/70088 A2 discloses a method of attaching unmodified biopolymers, particularly unmodified polynucleotides, directly to a solid support.

Situma et al., Analytical Biochemistry, Vol. 340; 2005; pp. 123-135 discloses the fabrication of DNA microarrays onto poly(methyl methacrylate) with ultraviolet patterning and microfluidics for the detection of low-abundant point mutations.

Situma et al., Biomolecular Engineering, Vol. 23 (5); 2006; pp. 213-231 provides an overview of the coupling of DNA, protein and cell microarrays to microfluidics and discusses potential improvements required for the implementation of these technologies into biomedical and clinical applications.

US5554501 A discloses organic polymers useful in the synthesis of biopolymers, the organic polymers being modified with an external chemical species, the modification being accomplished by means of the application of energy in the microwave or radio-frequency bands to the organic polymer in the presence of the external chemical species. In particular, oligonucleotides complementary to regions of genes of interest are synthesized onto the polymers, and these are in turn utilised for the analysis of patent samples.

US2001/018513 A1 discloses a method for extracting nucleic acids from a biological material such as blood, comprising contacting the mixture with a material at a pH such that the material is positively charged and will bind negatively charged nucleic acids, and then eluting the nucleic acids at a pH when the said materials possess a neutral or negative charge to release the nucleic acids.

Goli, "Functional Coatings Based on Denaturation and Adsorption of Proteins"; 2012; ISBN:978-1-267-50543-9 discloses how denatured protein molecules were physisorbed on hydrophobic surfaces including flat solid surfaces modified with self-assembled n-octadecyltrichlorosilane (ODTS) hydrophobic layers and polypropylene (PP) materials through nonspecific interactions followed by cross-linking the adsorbed proteins forming a stable coating.

For many applications, polycarbonate is now the preferred material for microfluidic applications involving biomaterials. The use of polycarbonate in microfluidic applications strikes a balance between flowability, and the ability to immobilise reagents on its surface.

Polypropylene is an inert, non-polar material which exhibits excellent flow characteristics. In addition, the low background fluorescence of polypropylene makes it well suited for techniques involving fluorescence detection. However, the inherent inert nature of polypropylene makes immobilisation on its surface difficult, thereby limiting its use in assays where attachment of reagents such as biomolecules, is often required. Hybridisation assays, for instance, typically require the flow of a medium over an immobilised single-stranded oligonucleotide, such as single-stranded DNA (ssDNA). Immobilisation of the ssDNA must be sufficiently stable to ensure that the strands of DNA remain attached to the surface during the performance of the assay, and must allow selective and sensitive detection in a reproducible manner. In addition, the immobilisation technique itself should be efficient and not require vast quantities or excesses of expensive materials, such as labelled biomolecules. To date, such immobilisation has proven challenging.

"Oligonucleotide" means a molecule which comprises a chain of nucleotides. Oligonucleotides are commonly in the form of DNA or ribonucleic acid (RNA). The term "oligonucleotide" includes polynucleotides of genomic DNA or RNA, complementary DNA (cDNA), and polynucleotides of semisynthetic, or synthetic origin. Standard nucleotide bases may also be substituted with nucleotide isoform analogs. In this context the term "oligonucleotide" is somewhat interchangeable with "polynucleotide" although generally refers to nucleotide chains of shorter length.

It is an object of the present invention to obviate or mitigate one or more of these problems, and in aspects of the invention, to provide an improved method of immobilising an oligonucleotide onto a polypropylene surface.

### Summary of the Invention

The present invention relates to a method of immobilizing an oligonucleotide on a polypropylene surface, the method comprising oxidising at least a portion of the polypropylene surface; and bringing an amine-terminated oligonucleotide into contact with the oxidised polypropylene surface in the presence of a carbodiimide coupling agent to immobilise the oligonucleotide to the oxidised polypropylene surface via the formation of amide bonds.

The invention also relates to a microfluidic cassette or portion thereof, e.g. a plug insertable to form part of a microfluidic channel, having an oligonucleotide immobilised thereon by the method of the invention.

Various further features and aspects of the invention are defined in the claims.

### Brief Description of the Drawings

Embodiments of the present invention will now be described by way of example only with reference to the accompanying drawings where like parts are provided with corresponding reference numerals and in which:
Figure 1 illustrates the reaction of an amine-terminated oligonucleotide with an activated polypropylene surface in the presence of a carbodiimide coupling agent;
Figure 2 shows images of three hybridised array plugs imaged at 200 ms;
Figure 3 shows fluorescence analysis of a standard DNA hybridisation assay using ssDNA immobilised on polypropylene according to the invention, without the optional blocking step (i), and with the optional blocking step (ii), at 600 ms (FIG. 3A) and at 800 ms (FIG. 3B).

### Detailed Description

The present invention relates to a method of immobilizing an oligonucleotide on a polypropylene surface, the method comprising oxidising at least a portion of the polypropylene surface; and bringing an amine-terminated oligonucleotide into contact with the oxidised polypropylene surface in the presence of a carbodiimide coupling agent to immobilise the oligonucleotide to the oxidised polypropylene surface via the formation of amide bonds.

According to the present invention, at least a portion of the polypropylene surface is oxidised. The polypropylene surface may be, for example, an internal or external surface of a cassette, slide, plate or any component of a microfluidic assay. As would be understood by one skilled in the art, in this embodiment, immobilisation of the oligonucleotide onto a portion of the surface only would be required, in order to allow the reagent to flow over the polypropylene surface and bind or react selectively with the immobilised oligonucleotide, such as in a hybridisation assay.

In an embodiment, the oxidising step is carried out using plasma activation, flame treatment, chemical oxidation or UV-ozone.

The plasma activation may be arc discharge, corona discharge, dielectric barrier discharge or piezoelectric direct discharge.

In an embodiment, the oxidising step is carried out using a low-pressure oxygen plasma system.

The parameters for performing the low-pressure oxygen plasma would be readily ascertained by one skilled in the art; however typically the process is carried out for from 1 minute to 15 minutes, with 70% to 100% generator power and from 2 sccm to 20 sccm, or from 2 sccm to 10 sccm oxygen, typically 5 sccm.

Typically, the low-pressure oxygen plasma activation generates multiple oxygen-containing groups such as hydroxyl, aldehyde and carboxylic acid groups, on the polypropylene surface. The carboxylic acid groups can react with the amine-terminated oligonucleotide in the presence of the carbodiimide coupling agent to form amide bonds. Advantageously, when oxygen plasma is used for functionalisation of the polypropylene surface, typically only oxygen-containing functional groups are generated, thereby minimising undesirable side-reactions which can result from the generation of other functional groups, such as nitrogen-containing groups which can result from ammonia plasma functionalisation. Oxygen plasma is also readily available and inexpensive, making it particular suitable for use in the method of the present invention.

Alternatively, the oxidising step can be carried out using chemical oxidation. Suitable chemical oxidation systems include chromium oxide, sodium dichromate, sulphuric acid, fuming nitric acid, potassium permanganate solution, ammonium peroxydisulphate solution, acid piranha solution, base piranha solution, methanol/hydrochloric acid; and combinations thereof.

In an embodiment, chemical oxidation can be performed using sodium dichromate and sulphuric acid.

Oxidation using acid etching leads to a high concentration of acid groups on the oxidised surface, making it particularly useful for the process of the present invention in which the carbodiimide coupling agent reacts with the acid groups, to form the active ester.

According to the invention, the amine-terminated oligonucleotide is brought into contact with the oxidised propylene surface. The amine-terminated oligonucleotide may be a labelled or unlabelled oligonucleotide.

In an embodiment, the oligonucleotide is single-stranded DNA (ssDNA).

In an embodiment, the ssDNA is a labelled DNA probe. Optionally, the labelled DNA probe is a fluorescent-labelled DNA probe.

For instance, the immobilised oligonucleotide can be amine-terminated ssDNA which can be incorporated in a hybridisation assay as a probe for target DNA. The ssDNA can be labelled, such as, for example, with a fluorescent cyanine dye, or a ruthenium dye. Polypropylene has low background fluorescence, and therefore the immobilisation of fluorescent labelled biomolecules to a polypropylene surface is particularly advantageous.

The terminal amine group may be a 5' amine group or a 3' amine group. In an embodiment, the terminal amine group is a 5' amine group. Amine-terminated oligonucleotides can be purchased commercially or can be prepared using known methods.

According to the invention, the amine-terminated oligonucleotide is brought into contact with the oxidised polypropylene surface in the presence of a carbodiimide coupling agent to immobilise the oligonucleotide to the oxidised polypropylene surface via the formation of amide bonds.

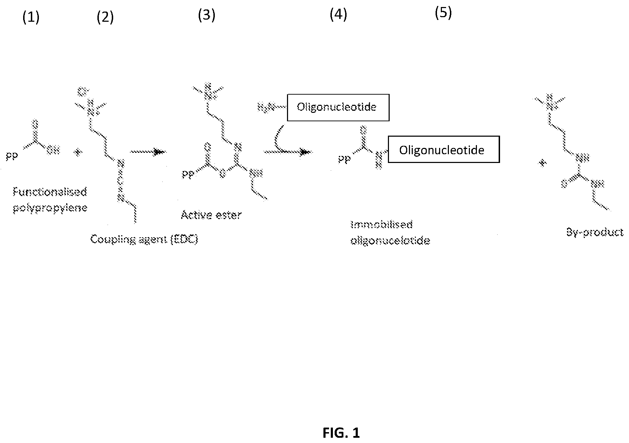

A carbodiimide coupling agent is a coupling agent comprising an RN=C=NR functional group. In a coupling reaction, the carbodiimide functional group activates a carboxylic acid group to form an activated carboxylic ester intermediate, which reacts with an amine group to yield an amide and urea. A method of immobilising an oligonucleotide on a polypropylene surface according to the method of the present invention is illustrated schematically in Figure 1, and shows reaction of an activated PP surface (1) with a carbodiimide coupling agent (in the exemplary schematic shown, this is EDC) (2) to yield an o-acylisourea active ester intermediate (3) and reaction of the o-acylisourea active ester intermediate (3) with amine-terminated ssDNA (4) to give immobilised ssDNA on the PP surface (5) via the formation of amide bonds, and an isourea by-product.

Carbodiimide coupling agents are known in the art and include, for example, 1-ethyl-3-(3-dimethylaminopropyl)carbodiimide (EDC); dicyclohexylcarbodiimide (DCC); diisopropylcarbodiimide (DIC); phenyl isopropyl carbodiimide (PIC); phenyl ethyl carbodiimide (PEC); *N*-tert-butyl-*N*'-methylcarbodiimide (BMC), *N*-*tert*-butyl-*N*'-ethylcarbodiimide (BEC), and bis[[4-(2,2-dimethyl-1,3-dioxolyl)]methyl]-carbodiimide (BDDC).

In an embodiment of the invention, the carbodiimide coupling agent is 1-ethyl-3-(3-dimethylaminopropyl)carbodiimide (EDC, but also referred to in the art as EDAC or EDCI).

The step of bringing the amine-terminated oligonucleotide into contact with the oxidised polypropylene surface in the presence of a carbodiimide coupling agent is carried out under conditions sufficient to immobilise the amine-terminated oligonucleotide via the formation of amide bonds with oxygen-containing functional groups on the activated polypropylene surface.

Typically, the contacting step is performed in the presence of a buffer.

Suitable buffers would be known to one skilled in the art and include, for example, 2-(*N-*morpholino)ethanesulfonic acid (MES), 3-(*N*-morpholino)propanesulfonic acid (MOPS), saline-sodium citrate (SSC), phosphate buffers, 2,2-Bis(hydroxymethyl)-2,2',2"-nitrilotriethanol (BIS-TRIS), piperazine-N,N'-bis(2-ethanesulfonic acid) (PIPES), 3-morpholinopropanesulfonic acid (MOPSO), 1,3-Bis[tris(hydroxymethyl)methylamino]propane (BIS-TRIS propane), N,N-Bis(2-hydroxyethyl)-2-aminoethanesulfonic acid, N,N-Bis(2-hydroxyethyl)taurine (BES) and 3-[(3-Cholamidopropyl)dimethylammonio]-1-propanesulfonate hydrate (CHAPS).

In an embodiment, the buffer is 2-(*N*-morpholino)ethanesulfonic acid (MES).

In an embodiment, the contacting step is performed by printing the amine-terminated oligonucleotide onto the oxidised polypropylene surface. The printed surface may be allowed to incubate for a period of from less than 1 hour to 14 days, e.g. from 5 minutes to 14 days, from 5 minutes to 10 days. Preferably the incubation is allowed to take place for from 5 minutes to 24 hours. Incubation may take place under ambient temperature and humidity conditions. If a fluorescent probe is being used, the printed surfaces may be kept in darkness during any incubation period.

In an embodiment of the invention, the polypropylene surface is an internal surface of a microfluidic channel in a microfluidic cassette for use in a hybridisation array or a surface of a plug/insert that forms part of a microfluidic channel. The ssDNA-immobilised PP surface may be used in a hybridisation assay.

It will be understood that the immobilisation of an oligonucleotide onto the internal surface can occur during the fabrication of a microfluidic cassette.

In an embodiment, the method further comprises the step of blocking the oligonucleotide-immobilised surface with a blocking agent.

According to this embodiment of the invention, non-specific binding of other biomolecules or other components of the assay to the activated polypropylene surface during performance of the assay can be minimised by blocking unoccupied binding sites with a blocking reagent. Any suitable blocking reagent, i.e. one which can reduce or eliminate non-specific binding, which does not participate in the reactions that form part of the assay, and which does not otherwise damage components of the assay, can be used.

Blocking agents which could be used include alkali and alkaline earth metal borohydrides such as sodium borohydride and lithium borohydride; aluminium borohydride, zinc borohydride, sodium cyanoborohydride, sodium triacetoxyborohydride, and acidic solutions of Zn²⁺ or Fe²⁺.

In an embodiment, the blocking agent is sodium borohydride.

In an embodiment, the method further comprises one or more washing steps. The washing steps may be performed prior to the blocking step. A detergent, such as sodium dodecyl sulfate (SDS) may be used in the washing step.

### Examples:

The following examples are intended to be illustrative only and are not intended to limit the scope of the invention.

### Example 1.1: Amine-terminated ssDNA

Unlabelled DNA probes modified on the 5' end with an amine termination, a C₁₂ linker and 2 hexaethylene glycol spacer modifications were purchased from Integrated DNA Technologies (Illinois, USA).

Cy5 and Cy3 labelled DNA probes, modified on the 5' end with an amine termination and a C₆ linker were purchased from Integrated DNA Technologies (Illinois, USA).

### Example 1.2 Preparation of DNA ink

A DNA ink for deposition was prepared as follows:

### 1.2.1 Preparation of MES buffer

A 0.1 M MES (w/0.9% NaCl, pH 4.6) buffer solution was prepared as follows: 1.066 g of MES monohydrate was weighed into a 50 ml falcon tube. 45 ml of ultrapure water was added from a measuring cylinder. 0.450 g of NaCl was weighed in a weighing boat and added to the MES solution, which was shaken until complete dissolution occurred. The pH of the solution was measured, and adjusted to pH 4.6 using a 0.1 M or 1 M NaOH solution prepared from NaOH pellets (Sigma-Aldrich, product # 221465). The remaining volume of ultrapure water needed to make up to 50 ml was added via pipette. The final pH was measured to confirm it fell within the range of pH 4.55 and pH 4.65. The solution was filtered through a 0.22 µm filter into a clean falcon tube. The buffer was stored in a fridge before use (up to 1 week maximum).

### 1.2.2 Preparation of coupling agent solution:

### 1.2.2.1. 20 mM EDC in 0.1 M MES buffer

A 20 mM EDC solution in 0.1 M MES buffer was prepared as follows: 3.83 mg of EDC was dissolved in 1 mL of the 0.1 M MES buffer prepared in example 1.2.1 above, in a glass sample vial. The solution was used within 10 hours of its preparation.

### 1.2.2.2 17.78 mM EDC in 0.089 M MES buffer containing 1.11% glycerol

When the solution was required, 160 µL of 20 mM EDC in 0.1 M MES buffer (section 1.2.2.1) was pipetted into an Eppendorf tube. 2 µL of glycerol was pipetted into the solution, followed by 18 µL of ultrapure water which was pipetted into the solution.

### 1.2.3 Preparation of DNA Ink

### 1.2.3.1 100 µM (unlabelled) DNA probe solution (1% glycerol)

A 20 µl 100 µM (unlabelled) DNA probe solution was prepared as follows:
18 µl of the 17.78 mM EDC in 0.089 M MES buffer with 1.11% glycerol solution prepared in section 1.2.2.2 was pipetted into an Eppendorf tube. 2 µl of 1 mM unlabelled DNA probe was pipetted into the solution.

### 1.2.3.2 50 µM (cyanine-labelled) DNA probe solution (1% glycerol)

A 20 µl 50 µM (Cy5 or Cy3 labelled) DNA probe solution was prepared as follows:
18 µl of the 17.78 mM EDC in 0.089 M MES buffer with 1.11% glycerol solution prepared in section 1.2.2.2 was pipetted into an Eppendorf tube. 1 µl of ultrapure water was pipetted into the solution and then 1 µl of 1 mM Cy5 or Cy3-labelled DNA probe was pipetted into the solution.

### Example 1.3: Activation of polypropylene surface

### 1.3.1 Pre-cleaning of PP surface

Polypropylene (PP) slides and polypropylene array plugs were rinsed with isopropyl alcohol (IPA) from a squeezy bottle at a minimum of 1 ml IPA per cm² of PP surface, and then dried using a stream of nitrogen until all visible traces of IPA had disappeared. The slides were then inserted into a glass holder and baked in the oven at 70 °C, atmospheric pressure, for 15 minutes.

Although the PP surface was pre-cleaned in this specific example, the cleaning step is optional, and subsequent testing has confirmed that it is not an essential step.

### 1.3.2 Activation of polypropylene surface

The clean polypropylene samples (slides and array plugs) from example 1.3.1 were placed into a plasma asher (Diener^{™} Pico, Serial number 116299) with the PP surface to which immobilisation is required facing upwards. The plasma asher generator power was set to 90%, the O₂ flow rate to 5 sccm, and the PP surface was activated for 15 minutes. Samples were then removed from the asher and placed in the printer chamber (Scienion SX, Berlin, Germany) until needed. All activated samples were used within 10 hours of activation.

### Example 1.4 DNA Deposition

10 minutes after plasma activation, the DNA ink solutions prepared in example 1.2 above were deposited onto the activated PP surfaces using a Scienion sciFLEXARRAYER SX printer (Scienion SX, Berlin, Germany). The humidity was set to 65% (cold mist), and the inks were printed with 2 drops per spot with a drop volume of between 200 pl and 220 pl. Immediately after deposition, the polypropylene slides and plugs were placed into a clean dish or tray, wrapped in foil, and left to incubate at ambient temperature and humidity for between 6 and 8 days.

### Example 1.5 Blocking

A blocking solution was prepared by adding 15 ml 10x phosphate-buffered saline (PBS), 135 ml ultrapure water and 50 ml ethanol to a 500 ml Duran bottle. 0.5 g of sodium borohydride (Sigma, product code 213462) was added to the solution. The solution was stirred for 15 minutes.

Five reservoirs were prepared with 1) 0.2% SDS, 2) ultrapure water; 3) the blocking solution prepared above; 4) 0.2% SDS and 5) ultrapure water. Immobilised slides and plugs prepared above were placed into the first reservoir, left for 2 minutes, then agitated by lifting up and down before being left in the reservoir for a further 2 minutes. The slides and plugs were then placed into the second reservoir containing clean ultrapure water, in a similar fashion (i.e. being left for 2 minutes, agitated, then left for 2 minutes). The slides and plugs were then placed into the third reservoir containing the blocking solution where they were left for 15 minutes. The slides and plugs were then placed into the fourth solution, i.e. a clean 0.2% SDS solution where they were left for 2 minutes, then agitated by lifting up and down, before being left in the reservoir for two minutes, and then they were placed into the fifth reservoir, which contained fresh ultrapure water, where they were again left for 2 minutes, then agitated by lifting up and down, before being left in the reservoir for two minutes. The slides and plugs were then dried using nitrogen. The slides or plugs were not allowed to dry between each step, and clean solutions were used for each reservoir.

### Example 1.6 Warfarin assay

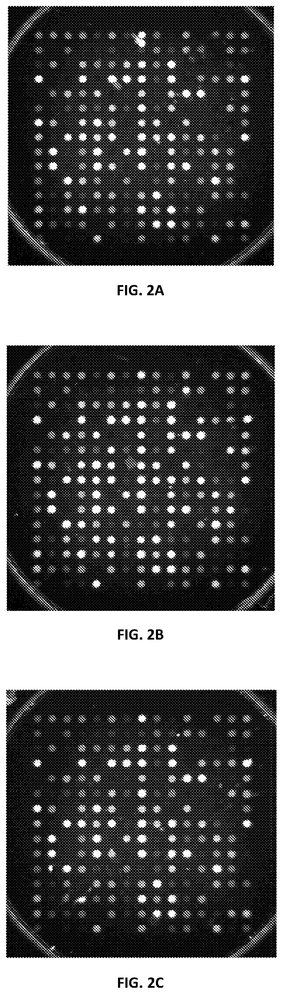

A warfarin assay was printed using the procedure outlined above. Three array plugs were hybridised with a PCR amplified swab sample. This swab sample is wild type homozygous in the *2 SNP and heterozygous in the *3 and VK SNPs. Figure 2 shows images of the three hybridised array plugs at 200 ms exposure, and fluorescence values are shown in Table 1. Discrimination factors were calculated and were in the ranges expected for a *2 wild type, *3 heterozygous, VK heterozygous sample.

**Table 1: median fluorescence intensity of each probe type on each of the three arrays**

| | Array plug 1 | Array plug 2 | Array plug 3 |
|---|---|---|---|
| Alignment control | 52,360 | 52,593 | 53,626 |
| CYP2C9*2 wildtype | 27,910 | 39,070 | 27,273 |
| CYP2C9*2 mutant | 695 | 1,574 | 885 |
| CYP2C9*3 wildtype | 19,037 | 25,319 | 15,556 |
| CYP2C9*3 mutant | 4,860 | 6,630 | 4,041 |
| VKORC1 wildtype | 56,616 | 56,541 | 56,142 |
| VKORC1 mutant | 31,738 | 43,516 | 25,477 |
| Non-specific control | -780 | -536 | -353 |

### Example 1.7 Hybridisation assay

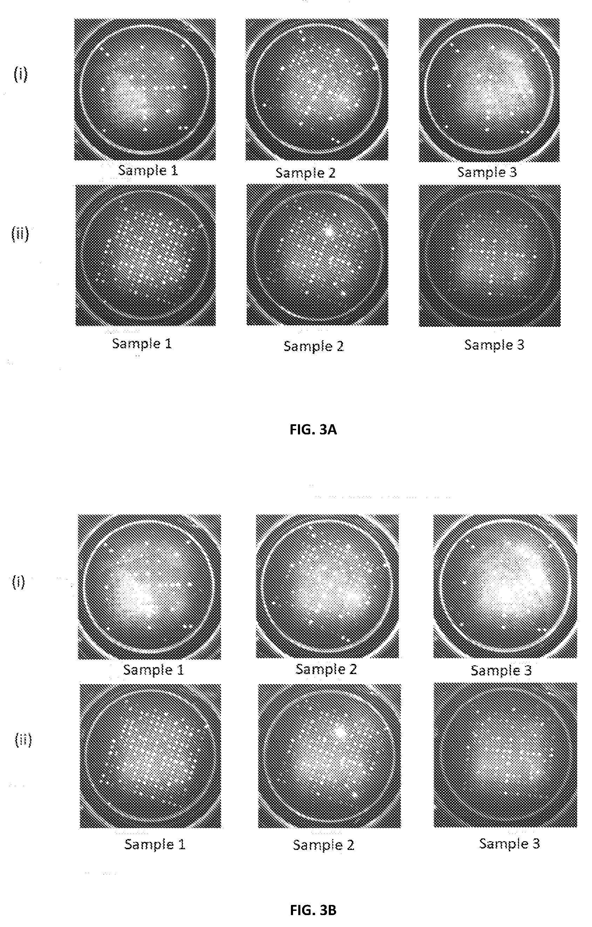

A standard DNA hybridisation assay was carried out using ssDNA immobilised on polypropylene according to the invention, with cyanine-labelled ssDNA used as a control. Tests were performed both for immobilised ssDNA which had not subsequently been subjected to a blocking step, and for immobilised ssDNA which had been subjected to a blocking step after 7 days incubation as outlined in Example 1.6. Measurements were taken at two exposure times, 600 ms and 800 ms. The results of this analysis are shown in Figure 3.

The experimental results demonstrate that the immobilisation technique according to the invention allows for selective and sensitive detection to take place, with minimal background signal for fluorescence measurements. The optional blocking step results in increased clarity for the microarray, as evidenced by the microarray spots in Figure 3.

The method of the invention therefore represents a convenient technique for the immobilisation of amine-terminated oligonucleotides onto polypropylene, using readily available instrumentation and reagents, and which results in sufficient reproducible immobilisation for use in hybridisation techniques.

## Claims

1. A method of immobilizing an oligonucleotide on a polypropylene surface, the method comprising:
oxidising at least a portion of the polypropylene surface;
and
bringing an amine-terminated oligonucleotide into contact with the oxidised polypropylene surface in the presence of a carbodiimide coupling agent to immobilise the oligonucleotide to the oxidised polypropylene surface via the formation of amide bonds.

2. A method as claimed in any preceding claim, wherein the oxidising step is carried out using plasma activation, flame treatment, chemical oxidation or UV-ozone.

3. A method as claimed in claim 2, wherein the oxidising step is carried out using oxygen plasma.

4. A method as claimed in any preceding claim, wherein the oligonucleotide is single-stranded DNA (ssDNA).

5. A method as claimed in claim 4, where the ssDNA is a labelled DNA probe, optionally wherein the labelled DNA probe is a fluorescent-labelled DNA probe.

6. A method as claimed in any preceding claim, wherein the carbodiimide coupling agent is 1-ethyl-3-(3-dimethylaminopropyl)carbodiimide (EDC), dicyclohexylcarbodiimide (DCC), diisopropylcarbodiimide (DIC), phenyl isopropyl carbodiimide (PIC), phenyl ethyl carbodiimide (PEC), *N*-*tert*-butyl-*N*'-methylcarbodiimide (BMC), N-tert-butyl-N'-ethylcarbodiimide (BEC), or bis[[4-(2,2-dimethyl-1,3-dioxolyl)]methyl]-carbodiimide (BDDC).

7. A method as claimed in any preceding claim, wherein the step of bringing the amine-terminated oligonucleotide into contact with the oxidised polypropylene surface is performed in the presence of a buffer.

8. A method as claimed in claim 8, wherein the buffer is selected from 2-(*N*-morpholino)ethanesulfonic acid (MES), 3-(*N*-morpholino)propanesulfonic acid (MOPS),saline-sodium citrate (SSC), 2,2-Bis(hydroxymethyl)-2,2',2"-nitrilotriethanol (BIS-TRIS), piperazine-N,N'-bis(2-ethanesulfonic acid) (PIPES), 3-morpholinopropanesulfonic acid (MOPSO), 1,3-Bis[tris(hydroxymethyl)methylamino]propane (BIS-TRIS propane), N,N-Bis(2-hydroxyethyl)-2-aminoethanesulfonic acid, N,N-Bis(2-hydroxyethyl)taurine (BES) and 3-[(3-Cholamidopropyl)dimethylammonio]-1-propanesulfonate hydrate (CHAPS) and phosphate buffers.

9. A method as claimed in any preceding claim, wherein the polypropylene surface is a surface of a plug or cassette for a hybridisation array, or a surface of a slide.

10. A method as claimed in any preceding claim, wherein the method further comprises the step of blocking the oligonucleotide-immobilised surface with a blocking agent.

11. A method as claimed in claim 10, wherein the blocking agent is sodium borohydride.

## Patentansprüche

1. Verfahren zur Immobilisierung eines Oligonukleotids auf einer Polypropylenoberfläche, das Verfahren umfassend:
Oxidieren mindestens eines Abschnitts der Polypropylenoberfläche;
und
Inkontaktbringen eines Oligonukleotidd mit Aminendgruppen in Gegenwart eines Carbodiimid-Kupplungsmittels mit der oxidierten Polypropylenoberfläche, um das Oligonukleotid durch die Bildung von Amidbindungen an der oxidierten Polypropylenoberfläche zu immobilisieren.

2. Verfahren nach einem der vorstehenden Ansprüche, wobei der Oxidationsschritt unter Verwendung von Plasmaaktivierung, Flammbehandlung, chemischer Oxidation oder UV-Ozon durchgeführt wird.

3. Verfahren nach Anspruch 2, wobei der Oxidationsschritt unter Verwendung von Sauerstoffplasma durchgeführt wird.

4. Verfahren nach einem der vorstehenden Ansprüche, wobei das Oligonukleotid einzelsträngige DNA (ssDNA) ist.

5. Verfahren nach Anspruch 4, wobei die ssDNA eine markierte DNA-Sonde ist, wobei die markierte DNA-Sonde gegebenenfalls eine fluoreszenzmarkierte DNA-Sonde ist.

6. Verfahren nach einem der vorstehenden Ansprüche, wobei das Carbodiimid-Kupplungsmittel 1-Ethyl-3-(3-dimethylaminopropyl)carbodiimid (EDC), Dicyclohexylcarbodiimid (DCC), Diisopropylcarbodiimid (DIC), Phenylisopropylcarbodiimid (PIC), Phenylethylcarbodiimid (PEC), *N*-tert-Butyl-*N*'-methylcarbodiimid (BMC), *N*-tert-Butyl-N'-ethylcarbodiimid (BEC) oder Bis[[4-(2,2-dimethyl-1,3-dioxolyl)]methyl]-carbodiimid (BDDC) ist.

7. Verfahren nach einem der vorstehenden Ansprüche, wobei der Schritt des Inkontaktbringens des Oligonukleotids mit Aminendgruppen mit der oxidierten Polypropylenoberfläche in Gegenwart eines Puffers durchgeführt wird.

8. Verfahren nach Anspruch 8, wobei der Puffer ausgewählt ist aus 2-(*N*-Morpholino)ethansulfonsäure (MES), 3-(*N*-Morpholino)propansulfonsäure (MOPS), Natriumcitrat-Kochsalzlösung (SSC), 2,2-Bis(hydroxymethyl)-2,2',2"-nitrilotriethanol (BIS-TRIS), Piperazin-N,N'-bis(2-ethansulfonsäure) (PIPES), 3-Morpholinopropansulfonsäure (MOPSO), 1,3-Bis[tris(hydroxymethyl)methylamino]propan (BIS-TRIS-Propan), N,N-Bis(2-hydroxyethyl)-2-aminoethansulfonsäure, N,N-Bis(2-hydroxyethyl)taurin (BES) sowie 3-[(3-Cholamidopropyl)dimethylammonio]-1-propansulfonat-Hydrat (CHAPS) und Phosphatpuffer.

9. Verfahren nach einem der vorstehenden Ansprüche, wobei die Polypropylenoberfläche eine Oberfläche eines Plugs oder einer Kassette für ein Hybridisierungsarray oder eine Oberfläche eines Objektträgers ist.

10. Verfahren nach einem der vorstehenden Ansprüche, wobei das Verfahren ferner den Schritt des Blockierens der mit Oligonukleotiden immobilisierten Oberfläche mit einem Blockierungsmittel umfasst.

11. Verfahren nach Anspruch 10, wobei das Blockierungsmittel Natriumborhydrid ist.

## Revendications

1. Procédé d'immobilisation d'un oligonucléotide sur une surface en polypropylène, le procédé comprenant :
l'oxydation d'au moins une partie de la surface en polypropylène ;
et
la mise en contact d'un oligonucléotide à terminaison amine avec la surface en polypropylène oxydée en présence d'un agent de couplage carbodiimide pour immobiliser l'oligonucléotide sur la surface en polypropylène oxydée par le biais de la formation de liaisons amides.

2. Procédé selon l'une quelconque revendication précédente, dans lequel l'étape d'oxydation est réalisée à l'aide d'une activation par plasma, d'un traitement à la flamme, d'une oxydation chimique ou d'un traitement UV-ozone.

3. Procédé selon la revendication 2, dans lequel l'étape d'oxydation est réalisée à l'aide d'un plasma d'oxygène.

4. Procédé selon l'une quelconque revendication précédente, dans lequel l'oligonucléotide est un ADN simple brin (ADNsb).

5. Procédé selon la revendication 4, dans lequel l'ADNsb est une sonde d'ADN marquée, éventuellement dans lequel la sonde d'ADN marquée est une sonde d'ADN marquée par fluorescence.

6. Procédé selon l'une quelconque revendication précédente, dans lequel l'agent de couplage carbodiimide est le 1-éthyl-3-(3-diméthylaminopropyl)carbodiimide (EDC), le dicyclohexylcarbodiimide (DCC), le diisopropylcarbodiimide (DIC), le phénylisopropylcarbodiimide (PIC), le phényléthylcarbodiimide (PEC), le *N*-tert-butyl-*N*'-méthylcarbodiimide (BMC), le *N*-tert-butyl-*N*'-éthylcarbodiimide (BEC), ou le bis[[4-(2,2-diméthyl-1,3-dioxolyl)]méthyl]-carbodiimide (BDDC).

7. Procédé selon l'une quelconque revendication précédente, dans lequel l'étape consistant à mettre en contact l'oligonucléotide à terminaison amine avec la surface en polypropylène oxydée est réalisée en présence d'un tampon.

8. Procédé selon la revendication 8, dans lequel le tampon est sélectionné parmi l'acide 2-(*N-*morpholino)éthanesulfonique (MES), l'acide 3-(*N-*morpholino)propanesulfonique (MOPS), la solution saline-citrate de sodium (SSC), le 2,2-bis(hydroxyméthyl)-2,2',2"-nitrilotriéthanol (BIS-TRIS), la pipérazine-N,N'-bis(acide 2-éthanesulfonique) (PIPES), l'acide 3-morpholinopropanesulfonique (MOPSO), le 1,3-bis[tris(hydroxyméthyl)méthylamino]propane (BIS-TRIS-propane), l'acide N,N-bis(2-hydroxyéthyl)-2-aminoéthanesulfonique, la N,N-bis(2-hydroxyéthyl)taurine (BES) et le 3-[(3-Cholamidopropyl)diméthylammonio]-1-propanesulfonate hydraté (CHAPS) et les tampons phosphate.

9. Procédé selon l'une quelconque revendication précédente, dans lequel la surface en polypropylène est une surface d'un bouchon ou d'une cassette destinée à une puce d'hybridation, ou une surface d'une lame.

10. Procédé selon l'une quelconque revendication précédente, dans lequel le procédé comprend en outre l'étape consistant à bloquer la surface avec oligonucléotides immobilisés avec un agent de blocage.

11. Procédé selon la revendication 10, dans lequel l'agent de blocage est le borohydrure de sodium.
